# EUROPÄISCHE PATENTSCHRIFT

(11) **EP 0 376 123 B1**
(45) Veröffentlichungstag und Bekanntmachung des Hinweises auf die Patenterteilung: **01.06.1994**
(21) Anmeldenummer: 89123440.3
(22) Anmeldetag: 19.12.1989
(51) Int. Cl.: C07D 201/08

(54) **Verfahren zur Herstellung von Caprolactam**
Process of preparation of caprolactam
Procédé de préparation de caprolactame

(30) Priorität: 24.12.1988 DE 3843791
(43) Veröffentlichungstag der Anmeldung: 04.07.1990
(73) Patentinhaber: BASF Aktiengesellschaft, 67063 Ludwigshafen (DE)
(72) Erfinder: Merger, Franz, Dr., D-6710 Frankenthal (DE); Fischer, Rolf, Dr., D-6900 Heidelberg (DE); Harder, Wolfgang, Dr., D-6940 Weinheim (DE); Priester, Claus-Ulrich, Dr., D-6700 Ludwigshafen (DE); Vagt, Uwe, Dr., D-6720 Speyer (DE)

(56) Entgegenhaltungen:
- EP-A- 0 023 751
- EP-A- 0 027 022
- EP-A- 0 234 295
- EP-A- 0 235 553
- EP-A- 0 271 815

## Beschreibung

Gegenstand der Erfindung ist ein Verfahren zur Herstellung von Caprolactam aus 5-Formylvaleriansäureestern.

Aus der DE-A- 36 02 376 (=EP-A-0234295) ist ein Verfahren zur Herstellung von Caprolactam bekannt, bei dem man 5-Formylvaleriansäureester mit überschüssigem Ammoniak und Wasserstoff in Gegenwart von Hydrierkatalysatoren unter erhöhtem Druck bei einer Temperatur von 40 bis 130°C unter Mitverwendung von Alkanolen als Reaktionsmedium umsetzt, anschließend Ammoniak und Wasserstoff entfernt und dann das so erhaltene Reaktionsgemisch auf eine Temperatur von 150 bis 250°C erhitzt. Dieses Verfahren hat den Nachteil, daß die Ausbeuten verbesserungsbedürftig sind und darüber hinaus in der Cyclisierungsstufe der Umsatz von 6-Aminocapronsäureester zu wünschen übrig läßt, so daß größere Mengen des thermisch instabilen 6-Aminocapronsäureesters zurückgeführt werden müssen.

In der DE-A- 36 02 375 EP-A-0235553 wird ein Verfahren zur Herstellung von Caprolactam beschrieben, bei dem man 5-Formylvaleriansäureester mit überschüssigem Ammoniak und Wasserstoff in Gegenwart von Hydrierkatalysatoren unter erhöhtem Druck bei einer Temperatur von 40 bis 130°C unter Mitverwendung von Alkanolen als Reaktionsmedium umsetzt, Ammoniak und Wasserstoff entfernt, das so erhaltene Reaktionsgemisch bei erhöhter Temperatur mit Wasser unter gleichzeitigem Abtrennen von Alkanolen umsetzt und anschließend auf eine Temperatur von 150 bis 370°C unter Bildung von Caprolactam erhitzt. Dieses Verfahren hat den Nachteil, daß größere Mengen an Alkanolen durch Destillation abgetrennt werden müssen. Darüber hinaus sind die Ausbeuten verbesserungsbedürftig.

Schließlich wird in der DE-A- 36 02 377 ein Verfahren zur Herstellung von Caprolactam beschrieben, bei dem man 5-Formylvaleriansäureester zunächst mit Wasser in Gegenwart von Katalysatoren bei einer Temperatur von 30 bis 200°C zu 5-Formylvaleriansäure hydrolysiert, diese mit Ammoniak und Wasserstoff unter Mitverwendung von Lösungsmitteln in Gegenwart von Hydrierkatalysatoren unter erhöhtem Druck bei einer Temperatur von 50 bis 150°C zu 6-Aminocapronsäure umsetzt und die erhaltene Lösung von 6-Aminocapronsäure nach Abtrennen von Ammoniak und Katalysatoren auf eine Temperatur von 150 bis 370°C unter Bildung von Caprolactam erhitzt. Für eine technische Realisierung des Verfahrens sind die Ausbeuten verbesserungsbedürftig. Zudem bedarf dieses Verfahren einer zusätzlichen Hydrolysestufe.

EP-A- 0271815 beschreibt ein Verfahren zur Herstellung von Caprolaktam aus 6-Aminocapronsäureestern durch Erhitzen in aromatischen Kohlenwasserstoffen mit einem Siedepunkt von 80 - 240°C bei 100 bis 320°C.

Es war deshalb die technische Aufgabe gestellt, ein Verfahren zur Herstellung von Caprolactam, ausgehend von 5-Formylvaleiansäureestern zur Verfügung zu stellen, das technisch einfach mit hohen Umsätzen und Selektivitäten sowie Raumzeitausbeuten verläuft und bei dem möglichst wenig Nebenprodukte gebildet werden.

Diese Aufgabe wird gelöst in einem Verfahren zur Herstellung von Caprolactam durch Umsetzen von 5-Formylvaleriansäureestern mit überschüssigem Ammoniak und Wasserstoff in Gegenwart von Hydrierkatalysatoren bei erhöhter Temperatur und unter erhöhtem Druck in einem Reaktionsmedium und Cyclisieren des erhaltenen 6-Aminocapronsäureesters in Gegenwart von Wasser bei erhöhter Temperatur, wobei man
a) 5-Formylvaleriansäureester mit flüssigem Ammoniak als Reaktionsmedium und Wasserstoff in Gegenwart von Ruthenium-Katalysatoren in flüssiger Phase bei einer Temperatur von 80 bis 140°C und unter einem Wasserstoffpartialdruck von 40 bis 1000 bar umsetzt
b) das Reaktionsmedium Ammoniak durch einen unter Reaktionsbedingungen inerten flüssigen aromatischen Kohlenwasserstoff mit einem Siedepunkt von 80 bis 240°C als Reaktionsmedium ersetzt.
c) das so erhaltene Gemisch in flüssiger Phase unter erhöhtem Druck auf eine Temperatur von 230-350°C unter Bildung von Caprolactam erhitzt und
d) aus dem so erhaltenen Reaktionsgemisch Caprolactam gewinnt.

Das neue Verfahren hat den Vorteil, daß auf die Mitverwendung von Alkanolen als Reaktionsmedium verzichtet werden kann. Weiter hat das neue Verfahren den Vorteil, daß in der Hydrierstufe hohe Katalysatorbelastungen erzielt und somit hohe Raumzeitausbeuten erreicht werden, wobei der Katalysator eine hohe Lebensdauer hat. Ferner hat das neue Verfahren den Vorteil, daß wenig Nebenprodukte anfallen.

Bevorzugte Ausgangsstoffe sind 5-Formylvaleriansäurealkylester, insbesondere solche von C₁- bis C₄-Alkanolen. Geeignete Ausgangsverbindungen sind z.B. 5-Formylvaleriansäuremethylester, -ethylester, -propylester, -isopropylester oder -n-butylester. Besondere technische Bedeutung hat 5-Formylvaleriansäuremethylester erlangt.

In der Stufe a wird die Umsetzung in flüssigem Ammoniak durchgeführt, wobei Ammoniak sowohl als Reaktionspartner als auch als Lösungsmittel wirkt. Im allgemeinen verwendet man je kg 5-Formylvaleriansäureester, 1 bis 6 kg, vorzugsweise 1,2 bis 3,6 kg, insbesondere 1,2 bis 2,4 kg Ammoniak. Die Umsetzung wird bei einer Temperatur von 80 bis 140°C, vorteilhaft von 100 bis 135°C, insbesondere von 110 bis 130°C, durchgeführt.

Vorteilhaft wird je Mol 5-Formylvaleriansäureester 1 bis 20 Mol Wasserstoff angewandt. Hierbei wird ein Wasserstoffpartialdruck von 40 bis 1000 bar, vorzugsweise 50 bis 500 bar, insbesondere 70 bis 200 bar eingehalten.

Erfindungsgemäß wird als Katalysator Ruthenium verwendet. Es ist möglich, Ruthenium in feiner Verteilung suspendiert anzuwenden. Vorzugsweise wird Ruthenium auf Trägern aufgebracht verwendet. Geeignete Träger sind beispielsweise Aluminiumoxid, Kieselgel, Titandioxid, Zirkondioxid, Magnesiumaluminate oder Magnesiumsilikate. Bevorzugt werden Aluminiumoxid oder Magnesiumaluminate, insbesondere γ-Aluminiumoxide als Träger verwendet. Ruthenium wird in an sich bekannter Weise durch Tränken der Träger mit wäßrigen Lösungen von Rutheniumsalzen, wie Rutheniumchlorid oder Rutheniumnitrat, und anschließendes Trocknen und gegebenenfalls Kalzinieren auf die Träger aufgebracht.

Die Ruthenium-Konzentration auf dem Träger beträgt in der Regel 0,1 bis 10 Gew.-%, vorzugsweise 0,5 bis 5 Gew.-%, insbesondere von 1 bis 3 Gew.-%. Die Aktivierung der Ruthenium-Trägerkatalysatoren erfolgt in der Regel im Wasserstoffstrom zweckmäßig bei einer Temperatur von 180 bis 250°C, insbesondere von 190 bis 230°C, z.B. innerhalb von 1 bis 20 h, vorzugsweise 1.5 bis 10 h.

In der Regel hält man eine Katalysatorbelastung von 0,1 bis 15 kg 5-Formylvaleriansäureester je kg Katalysator und Stunde ein. Besonders bewährt hat sich eine Katalysatorbelastung von 1 bis 10, insbesondere von 4 bis 10 kg 5-Formylvaleriansäureester je kg Katalysator und Stunde.

Die Umsetzung kann diskontinuierlich, z. B. in einem Hochdruckgefäß, durchgeführt werden. Vorteilhaft führt man die Umsetzung kontinuierlich, z. B. in einer Rührbehälterkaskade, z.B. 2 bis 5 Behältern durch. Vorteilhaft hat es sich erwiesen, wenn eine Rückvermischung während der Umsetzung vermieden wird. Besonders bewährt hat es sich deshalb, wenn ein Gemisch aus 5-Formylvaleriansäureester und Ammoniak zusammen mit Wasserstoff über einen in einer rohrförmigen Reaktionszone fest angeordneten Katalysator geleitet wird. Als besonders vorteilhaft hat sich hierbei die Sumpffahrweise erwiesen. Hierbei wird in einer im wesentlichen senkrecht angeordneten rohrförmigen Reaktionszone, z.B. mit einem Verhältnis von Länge : Durchmesser von 8 : 1 bis 50 : 1, in der der Katalysator fest angeordnet ist, von unten 5-Formylvalerainsäureester und flüssiger Ammoniak sowie Wasserstoff zugegeben und am Kopf der rohrförmigen Reaktionszone 6-Aminocapronsäureester sowie Ammoniak und Reaktionswasser und gegebenenfalls überschüssiger Wasserstoff entnommen.

Bei der bevorzugten kontinuierlichen Arbeitsweise ergibt sich die Verweilzeit in Stufe a aus der Katalysatorbelastung und dem Ammoniakangebot. Sie liegt vorteilhaft im Bereich von 0,5 bis 20 Minuten, vorzugsweise von 1 bis 10, insbesondere 2 bis 6 Minuten.

Das so erhaltene Reaktionsgemisch enthält nach Entspannen und Abtrennen des Wasserstoffs 6-Aminocapronsäureester, Ammoniak sowie Wasser, das bei der Reaktion entsteht, ferner Nebenprodukte wie Caprolactam in geringen Mengen.

In der Stufe b wird das Reaktionsmedium Ammoniak durch unter Reaktionsbedingungen flüssige inerte aromatische Kohlenwasserstoffe mit einem Siedepunkt von 80 bis 240°C als Reaktionsmedium ersetzt. Bevorzugte aromatische Kohlenwasserstoffe haben einen Siedepunkt von 110 bis 200°C. Bevorzugte aromatische Kohlenwasserstoffe sind Alkylbenzole, insbesondere solche, die 1 bis 3 Alkylgruppen mit bis zu 6 Kohlenstoffatomen enthalten. Besonders bevorzugt sind Alkylbenzole mit 1 bis 3 Alkylresten mit insgesamt bis zu 4 Kohlenstoffatomen. Geeignete aromatische Kohlenwasserstoffe sind beispielsweise Benzol, Toluol, Xylole, Ethylbenzol, Diethylbenzol, Trimethylbenzol, Isopropylbenzol, Propyl- oder Diisopropylbenzol. Besonders vorteilhaft sind Toluol und Xylole. Pro kg Aminocapronsäureester setzt man in der Regel 2 bis 20 kg aromatischen Kohlenwasserstoff ein. Vorteilhaft wendet man 4 bis 15 kg, insbesondere 5 bis 12 kg aromatische Kohlenwasserstoffe je kg 6-Aminocapronsäureester an.

In der Regel wird der Reaktionsaustrag aus der Stufe a vor dem Entspannen mit aromatischen Kohlenwasserstoffen in der angegebenen Menge versetzt, auf Normaldruck entspannt und hierbei Wasserstoff abgetrennt und dann überschüssiges Ammonaik abdestilliert. Dies erfolgt vorteilhaft durch Destillation z.B. durch Strippen in einer Kolonne unter Mitverwendung von Inertgasen wie Stickstoff. Das Abdestillieren des Ammoniaks kann auch unter erhöhtem Druck erfolgen. Nach einer anderen Arbeitsweise wird der Reaktionsaustrag aus der Stufe a zunächst entspannt und hierbei Wasserstoff abgetrennt und dann aromatische Kohlenwasserstoffe zugegeben und anschließend Ammoniak abdestilliert. Nach einer weiteren Möglichkeit wird das aus der Reaktionsstufe a erhaltene Gemisch zunäcnst entspannt und hierbei Wasserstoff abgetrennt, Ammoniak abdestilliert und das verbleibende Gemisch aus 6-Aminocapronsäureester und Wasser mit aromatischen Kohlenwasserstoffen gemischt.

In der Regel wird die Umsetzung in Stufe b in Gegenwart des Reaktionswassers, d.h. mit molaren Mengen Wasser, bezogen auf in Stufe a eingesetzten Formylvaleriansäureester, durchgeführt.

In der Stufe b wird vorzugsweise der Wassergehalt des Gemisches aus aromatischen Kohlenwasserstoffe 6-Aminocapronsäureester und dem Reaktionswasser aus der aminierenden Hydrierung durch Zugabe von zusätzlichem Wasser auf einen Wassergehalt von 1,1 bis 10 Mol Wasser je Mol 5-Formylvaleriansäureester, die in der Stufe a eingesetzt wurden, eingestellt. Vorteilhaft hält man einen Wassergehalt von 1,5 bis 6 Mol, insbesondere 2 bis 4 Mol Wasser je Mol eingesetzten 5-Formylvaleriansäureester ein. Die Zugabe des Wassers erfolgt vorteilhaft nach dem Abtrennen von Ammoniak. Nach einer vorteilhaften Arbeitsweise wird das Gemisch von 6-Aminocapronsäureestern, aromatischen Kohlenwasserstoffen und Wasser emulgiert, vorzugsweise bei einer Temperatur von -10 bis +20°C. Man erhält so ein Gemisch, das aromatische Kohlenwasserstoffe als Reaktionsmedium, 6-Aminocapronsäureester, Wasser und geringe Mengen an Caprolactam enthält. Eine typische Zusammensetzung ist beispielsweise 65 bis 95 Gew.-% aromatische Kohlenwasserstoffe 6 bis 20 Gew.-% 6-Aminocapronsäureester 2 bis 20 Gew.-% Wasser sowie bis zu 1 Gew.-% Caprolactam.

In der Stufe c wird das so erhaltene Gemisch in flüssiger Phase unter erhöhtem Druck auf eine Temperatur von 230 bis 350°C unter Bildung von Caprolactam erhitzt. Vorteilhaft erhält man eine Temperatur von 260 bis 340°C ein. Die Temperatur und Druckbedingungen werden so gewählt, daß das Reaktionsgemisch stets im flüssigen Zustand vorliegt. Vorteilhaft stellt man einen Druck von 30 bis 200 bar, insbesondere 40 bis 110 bar ein.

Vorzugsweise hält man bei der Umsetzung eine Verweilzeit von 5 bis 60 min, vorteilhaft 7 bis 45 min, insbesondere 10 bis 20 min, ein.

Die Mischung, enthaltend 6-Aminocapronsäureester, Wasser und aromatische Kohlenwasserstoffe, wird auf die angegebene Temperatur erhitzt, wobei man die Druck- und Temperaturbedingungen so wählt, daß das Reaktionsgemisch in flüssigem Zustand vorliegt. Die Umsetzung kann diskontinuierlich z.B. in Druckbehältern durchgeführt werden. Vorzugsweise führt man die Umsetzung kontinuierlich z.B. in Druckbehälterkaskaden, z.B. 2 bis 4 hintereinander geschalteten
Druckbehältern durch. Besonders bewährt hat es sich, wenn man die Umsetzung in einer rohrförmigen Reaktionszone, z.B. mit einem Verhältnis von Länge : Durchmesser von 100 zu 1 bis 1000 zu 1 durchführt.

In der Stufe d wird aus dem so erhaltenen Reaktionsgemisch, das aromatische Kohlenwasserstoffe, Caprolactam, Wasser, die den Estern entsprechenden Alkohole sowie geringe Mengen an 6-Aminocapronsäureestern enthält, Caprolactam gewonnen. In der Regel wird das erhaltene Reaktionsgemisch fraktioniert destilliert und Caprolactam isoliert. Die hierbei anfallenden aromatischen Kohlenwasserstoffe werden zweckmäßig wieder zurückgeführt. Nach einer bevorzugten Arbeitsweise wird Caprolactam aus den aromatischen Kohlenwasserstoffen mit Wasser extrahiert. Vorteilhaft wird die Extraktion im Gegenstrom in bekannten Vorrichtungen, z.B. Mixer Settler-Vorrichtungen, Rührscheibenkolonnen oder Siebbodenkolonnen mit oder ohne Pulsation durchgeführt. Vorteilhaft hält man bei der Extraktion eine Temperatur von 20 bis 80°C ein. Es hat sich ferner bewährt, wenn ein Teilstrom der zurückgewonnenen aromatischen Kohlenwasserstoffe vor der Wiederverwendung durch Destillation gereinigt wird.

Caprolactam, das nach dem Verfahren der Erfindung erhältlich ist, eignet sich zur Herstellung von Polycaprolactam.

Das Verfahren nach der Erfindung sei an folgenden Beispielen veranschaulicht.

### Beispiel 1

Ein vertikaler Rohrreaktor (Durchmesser: 9 mm, Füllhöhe: 37 cm, ölbeheizter Doppelmantel) wurde mit 17.2 g eines Katalysators mit 2,78 % Ruthenium auf Aluminiumoxid in Form von 1,5-mm-Strängen gefüllt (Katalysatorherstellung durch Diffusionstränkung mit wäßriger Rutheniumchlorid-Lösung und Trocknen bei 70°C) und zur Aktivierung des Katalysators unter Durchleiten von 20 Nl/h Wasserstoff die Temperatur innerhalb von 7 h von 100 auf 220°C erhöht und dann 6 h bei 220°C gehalten.

Nach Abkühlen auf 128°C wurden durch den Reaktor bei einem Druck von 98 bar stündlich von unten nach oben unter gleichzeitigem Durchleiten von 66 Nl/h (2,9 mol) Wasserstoff 100,8 ml 5-Formylvaleriansäuremethylester (Reinheit: 99 %; 102,2 g, 0,710 mol) und 280 ml (168 g, 9.9 mol) flüssiges Ammoniak gepumpt.

Der Reaktionsaustrag wurde über ein Druckhalteventil auf Normaldruck entspannt, in 1035 ml Xylol pro Stunde aufgenommen und auf den Kopf einer auf 40°C beheizten, 15 cm langen und mit 5 mm Glasringen gefüllten Kolonne geleitet, durch die im Gegenstrom 20 l/h Stickstoff geblasen wurden. Am Kolonnensumpf wurden stündlich 1019 g eines zweiphasigen Gemisches erhalten. Nach Homogenisierung einer Probe mit Methanol wurde durch quantitative gaschromatographische Analyse ein Gehalt von 8,6 % 6-Aminocapronsäuremethylester und 0.3 % Caprolactam bestimmt, entsprechend Hydrierausbeuten von 85,1 % 6-Aminocapronsäuremethylester bzw. 3,8 % Caprolactam.

Die beiden Phasen wurden getrennt und die wäßrige Phase (19,8 g/h) mit 26 g/h (1,4 mol) Wasser versetzt. In einen 70 ml Rohrreaktor (2,2 mm Durchmesser) wurden bei einem Druck von 100 bar und einer Temperatur von 270°C stündlich 8,25 ml (8,09 g) der wäßrigen Phase und 260 ml (228,9 g) xylolische Phase zudosiert.

Der Austrag aus 2,55 h wurde am Rotationsverdampfer eingeengt. Es verblieben 52,3 g Rückstand, der nach quantitativer gaschromatographischer Analyse 72,9 % Caprolactam und 3,0 % 6-Aminocapronsäuremethylester enthielt. Die Gesamtausbeute an Caprolactam beträgt 81,9 %, bezogen auf 5-Formylvaleriansäuremethylester.

### Beispiel 2

Ein vertikaler Rohrreaktor (Durchmesser: 9 mm, Füllhöhe: 37 cm, ölbeheizter Doppelmantel) wurde mit 14.0 g eines Katalysators mit 2.78 % Ruthenium auf Aluminiumoxid in Form von 1,5-mm-Strängen gefüllt (Katalysatorherstellung durch Diffusionstränkung mit wäßriger Rutheniumchlorid-Lösung und Trocknen bei 70°C) und zur Aktivierung des Katalysators unter Durchleiten von 60 Nl/h eines Stickstoff-Wasserstoff-Gemisches (5 : 1) die Temperatur innerhalb von 6 h von 20 auf 200°C erhöht und dann 6 h bei 200°C gehalten.

Nach Abkühlen auf 126°C wurden durch den Reaktor bei einem Druck von 99 bar stündlich von unten nach oben unter gleichzeitigem Durchleiten von 52 Nl/h (2,3 mol) Wasserstoff 85,6 ml 5-Formylvaleriansäuremethylester (87,7 g, 0,609 mol) und 368 ml (221 g, 13 mol) flüssiges Ammoniak gepumpt.

Der Reaktionsaustrag wurde über ein Druckhalteventil auf Normaldruck entspannt, in 336 ml Xylol pro Stunde aufgenommen und auf den Kopf einer auf 40°C beheizten, 15 cm langen und mit 5 mm Glasringen gefüllten Kolonne geleitet, durch die im Gegenstrom 20 l/h Stickstoff geblasen wurden. Am Kolonnensumpf wurden stündlich 376,6 g eines zweiphasigen Gemisches erhalten, das nach quantitativer gaschromatographischer Analyse 21,6 % 6-Aminocapronsäuremethylester und 0,6 % Caprolactam enthielt entsprechend Hydrierausbeuten von 92.2 % bzw. 3.3 %.

Das zweiphasige Gemisch wurde in einem auf 0°C thermostatisierten Doppelmantelkolben emulgiert und daraus stündlich 359,0 ml durch einen 70-ml-Rohrreaktor gepumpt, (2,2 mm Durchmesser) wobei ein Druck von 75 bar und eine Temperatur von 270°C eingehalten wurde. Der Austrag aus 1,83 h (750,1 g) wurde am Rotationsverdampfer eingeengt. Es verbleiben 104,2 g Rückstand, der nach quantitativer gaschromatographischer Analyse 90,1 % Caprolactam und 0,34 % 6-Aminocapronsäuremethylester enthielt. Die Gesamtausbeute an Caprolactam beträgt 84,4 %, bezogen auf 5-Formylvaleriansäuremethylester.

## Patentansprüche

1. Verfahren zur Herstellung von Caprolactam durch Umsetzen von 5-Formylvaleriansäureestern mit überschüssigem Ammoniak und Wasserstoff in Gegenwart von Hydrierkatalysatoren bei erhöhter Temperatur und unter erhöhtem Druck, in einem Reaktionsmedium und Cyclisieren des so erhaltenen 6-Aminocapronsäureesters in Gegenwart von Wasser bei erhöhter Temperatur, dadurch gekennzeichnet, daß man
a) 5-Formylvaleriansäureester mit flüssigem Ammoniak als Reaktionsmedium und Wasserstoff in Gegenwart von Ruthenium-Katalysatoren in flüssiger Phase bei einer Temperatur von 80 bis 140°C und unter einem Wasserstoffpartialdruck von 40 bis 1000 bar umsetzt.
b) das Reaktionsmedium Ammoniak durch unter Reaktionsbedingungen flüssige aromatische Kohlenwasserstoffe mit einem Siedepunkt von 80 bis 240°C als Reaktionsmedium ersetzt.
c) das so erhaltene Gemisch in flüssiger Phase unter erhöhtem Druck auf eine Temperatur von 230 bis 350°C unter Bildung von Caprolactam erhitzt und
d) aus dem so erhaltenen Reaktionsgemisch Caprolactam gewinnt.

2. Verfahren nach Anspruch 1, dadurch gekennzeichnet, daß man je kg 5-Formylvaleriansäureester 1,2 bis 3,6 kg Ammoniak anwendet.

3. Verfahren nach den Ansprüchen 1 bis 2, dadurch gekennzeichnet, daß man einen Trägerkatalysator mit 0,1 bis 10 Gew.-% Ruthenium verwendet.

4. Verfahren nach den Ansprüchen 1 bis 3, dadurch gekennzeichnet, daß man in der Stufe a eine Temperatur von 100 bis 135°C einhält.

5. Verfahren nach den Ansprüchen 1 bis 4, dadurch gekennzeichnet, daß man in der Stufe a eine Verweilzeit von 1 bis 10 min einhält.

6. Verfahren nach den Ansprüchen 1 bis 5, dadurch gekennzeichnet, daß man eine Katalysatorbelastung von 0,1 bis 15 kg 5-Formylvaleriansäureester je kg Katalysator und Stunde einhält.

7. Verfahren nach den Ansprüchen 1 bis 6, dadurch gekennzeichnet, daß man 5-Formylvaleriansäureester mit flüssigem Ammoniak und Wasserstoff über einen in einer rohrförmigen Reaktionszone fest angeordneten Ruthenium und Trägerkatalysator in Sumpffahrweise im wesentlichen unter Vermeidung von Rückvermischung leitet.

8. Verfahren nach den Ansprüchen 1 bis 7, dadurch gekennzeichnet, daß man in der Stufe b unter Reaktionsbedingungen flüssige aromatische Kohlenwasserstoffe mit einem Siedepunkt von 110 bis 200°C anwendet.

9. Verfahren nach den Ansprüchen 1 bis 8, dadurch gekennzeichnet, daß man je kg 6-Aminocapronsäureester 2 bis 20 kg aromatische Kohlenwasserstoffe verwendet.

10. Verfahren nach den Ansprüchen 1 bis 9, dadurch gekennzeichnet, daß man das Reaktionsgemisch aus der Stufe a mit aromatischen Kohlenwasserstoffen vermischt und Ammoniak abdestilliert.

11. Verfahren nach den Ansprüchen 1 bis 9, dadurch gekennzeichnet, daß man aus dem Reaktionsgemisch aus der Stufe a Ammoniak abdestilliert und das verbleibende Gemisch mit aromatischen Kohlenwasserstoffen mischt.

12. Verfahren nach den Ansprüchen 1 bis 11, dadurch gekennzeichnet, daß man in der Stufe b einen Wassergehalt von 1,1 bis 10 Mol-je Mol 5-Formylvaleriansäureester, bezogen auf die in der Stufe a eingesetzte Menge, einstellt.

13. Verfahren nach den Ansprüchen 1 bis 12, dadurch gekennzeichnet, daß man das aromatische Kohlenwasserstoffe, 6-Aminocapronsäureester und Wasser enthaltende Gemisch bei einer Temperatur von -10 bis +20°C emulgiert.

14. Verfahren nach den Ansprüchen 1 bis 13, dadurch gekennzeichnet, daß man in der Stufe d eine Temperatur von 260 bis 340°C einhält.

15. Verfahren nach den Ansprüchen 1 bis 14, dadurch gekennzeichnet, daß man in der Stufe c einen Druck von 40 bis 110 bar einhält.

16. Verfahren nach den Ansprüchen 1 bis 15, dadurch gekennzeichnet, daß man in der Stufe c eine Verweilzeit von 5 bis 60 min einhält.

17. Verfahren nach den Ansprüchen 1 bis 16, dadurch gekennzeichnet, daß man in der Stufe d Caprolactam durch Extraktion mit Wasser gewinnt.

## Claims

1. A process for preparing caprolactam by reacting a 5-formylvaleric ester with excess ammonia and hydrogen in the presence of a hydrogenation catalyst at elevated temperature under superatmospheric pressure in a reaction medium and cyclizing the resulting 6-aminocaproic ester in the presence of water at elevated temperature, comprising the steps of
a) reacting the 5-formylvaleric ester with liquid ammonia as reaction medium and hydrogen in the presence of a ruthenium catalyst in liquid phase at from 80 to 140°C under a hydrogen partial pressure of from 40 to 1000 bar,
b) replacing the reaction medium ammonia by an aromatic hydrocarbon having a boiling point of from 80 to 240°C which is liquid under the reaction conditions,
c) heating the resulting mixture in liquid phase under superatmospheric pressure at from 230 to 350°C to form caprolactam, and
d) isolating caprolactam from the resulting reaction mixture.

2. A process as claimed in claim 1, wherein from 1.2 to 3.6 kg of ammonia are used per kg of 5-formylvaleric ester.

3. A process as claimed in claim 1 or 2, wherein a supported catalyst containing from 0.1 to 10% by weight of ruthenium is used.

4. A process as claimed in any of claims 1 to 3, wherein a temperature of from 100 to 135°C is maintained in stage a.

5. A process as claimed in any of claims 1 to 4, wherein a residence time of from 1 to 10 minutes is maintained in stage a.

6. A process as claimed in any of claims 1 to 5, wherein a weight hourly space velocity of from 0.1 to 15 kg of 5-formylvaleric ester per kg of catalyst per hour is maintained.

7. A process as claimed in any of claims 1 to 6, wherein the 5-formylvaleric ester is passed with liquid ammonia and hydrogen over a fixed bed supported ruthenium catalyst in a tubular reaction zone in a liquid phase procedure with substantial avoidance of back mixing.

8. A process as claimed in any of claims 1 to 7, wherein an aromatic hydrocarbon having a boiling point of from 110 to 200°C which is liquid under reaction conditions is used in stage b.

9. A process as claimed in any of claims 1 to 8, wherein from 2 to 20 kg of aromatic hydrocarbons are used per kg of 6-aminocaproic ester.

10. A process as claimed in any of claims 1 to 9, wherein the reaction mixture of stage a is mixed with an aromatic hydrocarbon and ammonia is distilled off.

11. A process as claimed in any of claims 1 to 9, wherein ammonia is distilled out of the reaction mixture from stage a and the remaining mixture is mixed with an aromatic hydrocarbon.

12. A process as claimed in any of claims 1 to 11, wherein a water content of from 1.1 to 10 moles per mole of 5-formylvaleric ester, based on the amount used in stage a, is set in stage b.

13. A process as claimed in any of claims 1 to 12, wherein the mixture of aromatic hydrocarbon, 6-aminocaproic ester and water is emulsified at from -10 to +20°C.

14. A process as claimed in any of claims 1 to 13, wherein a temperature of from 260 to 340°C is maintained in stage d.

15. A process as claimed in any of claim 1 to 14, wherein a pressure of from 40 to 110 bar is maintained in stage c.

16. A process as claimed in any of claims 1 to 15, wherein a residence time of from 5 to 60 minutes is maintained in stage c.

17. A process as claimed in any of claims 1 to 16, wherein caprolactam is isolated by extraction with water in stage d.

## Revendications

1. Procédé de préparation de caprolactame par mise en réaction d'esters d'acide 5-formylvalérique avec de l'ammoniac en excès et de l'hydrogène en présence de catalyseurs d'hydrogénation à température élevée et sous pression élevée, dans un milieu de réaction, et cyclisation de l'ester d'acide 6-aminocaproïque ainsi obtenu en présence d'eau à température élevée, caractérisé en ce que
a) on met en réaction l'ester d'acide 5-formylvalérique avec de l'ammoniac liquide comme milieu réactionnel et de l'hydrogène en présence de catalyseurs au ruthénium en phase liquide à une température de 80 à 140°C et sous une pression partielle d'hydrogène de 40 à 1000 bar,
b) on remplace l'ammoniac du milieu réactionnel par des hydrocarbures aromatiques liquides dans les conditions de la réaction ayant un point d'ébullition de 80 à 240°C en tant que milieu réactionnel,
c) on chauffe le mélange ainsi obtenu en phase liquide fous pression élevée à une température de 230 à 350°C avec formation de caprolactame et
d) on récupère le caprolactame du mélange réactionnel ainsi obtenu.

2. Procédé selon la revendication 1, caractérisé en ce qu'on utilise 1,2 à 3,6 kg d'ammoniac par kg d'ester d'acide 5-formylvalérique.

3. Procédé selon la revendication 1 ou 2, caractérisé en ce qu'on utilise un catalyseur supporté avec 0,1 à 10% en poids de ruthénium.

4. Procédé selon l'une quelconque des revendications 1 à 3, caractérisé en ce qu'on maintient dans l'étape a une température de 100 à 135°C.

5. Procédé selon l'une quelconque des revendications 1 à 4, caractérisé en ce qu'on maintient dans l'étape a une durée de séjour de 1 à 10 minutes.

6. Procédé selon l'une quelconque des revendications 1 à 5, caractérisé en ce qu'on maintient une charge du catalyseur de 0,1 à 15 kg d'ester d'acide 5-formylvalérique par kg de catalyseur et par heure.

7. Procédé selon l'une quelconque des revendications 1 à 6, caractérisé en ce qu'on charge l'ester d'acide 5-formylvalérique avec l'ammoniac liquide et l'hydrogène sur un catalyseur supporté et du ruthénium disposés de manière fixe dans une zone réactionnelle tubulaire, de haut en bas en évitant essentiellement un mélange en retour.

8. Procédé selon l'une quelconque des revendications 1 à 7, caractérisé en ce qu'on utilise dans l'étape b des hydrocarbures aromatiques liquides dans les conditions réactionnelles avec un point d'ébullition de 110 à 200°C.

9. Procédé selon l'une quelconque des revendications 1 à 8, caractérisé en ce qu'on utilise de 2 à 20 kg d'hydrocarbures aromatiques par kg d'ester d'acide 6-aminocaproïque.

10. Procédé selon l'une quelconque des revendications 1 à 9, caractérisé en ce qu'on mélange le mélange réactionnel de l'étape a avec des hydrocarbures aromatiques et on élimine l'ammoniac par distillation.

11. Procédé selon l'une quelconque des revendications 1 à 9, caractérisé en ce qu'on élimine par distillation l'ammoniac à partir du mélange réactionnel de l'étape a et que l'on mélange le mélange restant avec des hydrocarbures aromatiques.

12. Procédé selon l'une quelconque des revendications 1 à 11, caractérisé en ce que l'on maintient dans l'étape b une teneur en eau de 1,1 à 10 moles par mole d'ester d'acide 5-formylvalérique, par rapport à la quantité introduite dans l'étape a.

13. Procédé selon l'une quelconque des revendications 1 à 12, caractérisé en ce qu'on émulsifie le mélange contenant des hydrocarbures aromatiques, de l'ester d'acide 6-aminocaproïque et de l'eau à une température de -10 à +20°C.

14. Procédé selon l'une quelconque des revendications 1 à 13, caractérisé en ce que l'on maintient dans l'étape d une température de 260 à 340°C.

15. Procédé selon l'une quelconque des revendications 1 à 14, caractérisé en ce qu'on maintient dans l'étape c une pression de 40 a 110 bar.

16. Procédé selon l'une quelconque des revendications 1 à 15, caractérisé en ce qu'on maintient dans l'étape c une durée de séjour de 5 à 60 minutes.

17. Procédé selon l'une quelconque des revendications 1 à 16, caractérisé en ce qu'on récupère dans l'étape d le caprolactame par extraction avec de l'eau.
